# EUROPEAN PATENT SPECIFICATION

(11) **EP 1 216 298 B1**
(45) Date of publication and mention of the grant of the patent: **13.06.2007**
(21) Application number: 00977269.0
(22) Date of filing: 27.09.2000
(51) Int. Cl.: C12N 15/00, A01N 63/00, A61K 48/00

(54) **METHODS OF PRODUCING NON-HUMAN CLONED AND TRANSGENIC MAMMALS**
VERFAHREN ZUR HERSTELLUNG VON NICHT-HUMANEN GEKLONTEN UND TRANSGENEN SÄUGETIEREN
PROCEDES DE PRODUCTION DE MAMMIFERES NON-HUMAINS CLONES ET TRANSGENIQUES

(30) Priority: 30.09.1999 US 157193 P
(43) Date of publication of application: 26.06.2002
(73) Proprietor: GTC Biotherapeutics, Inc., Framingham, MA 01702 (US)
(72) Inventor: ECHELARD, Yann, Jamaica Plains, MA 02131 (US)
(74) Representative: Ruffles, Graham Keith
(86) International application number: PCT/US2000/041007
(87) International publication number: WO 2001/023525

(56) References cited:
- EP-A- 0 930 009
- LUO J ET AL: "PLACENTAL ABNORMALITIES IN MOUSE EMBRYOS LACKING THE ORPHAN NUCLEARRECEPTOR ERR-BETA" NATURE, MACMILLAN JOURNALS LTD. LONDON, GB, vol. 388, 21 August 1997 (1997-08-21), pages 778-782, XP002938871 ISSN: 0028-0836
- NAGY A ET AL: "EMBRYONIC STEM CELLS ALONE ARE ABLE TO SUPPORT FETAL DEVELOPMENT IN THE MOUSE" DEVELOPMENT, COMPANY OF BIOLOGISTS, CAMBRIDGE,, GB, vol. 110, no. 3, 1 November 1990 (1990-11-01), pages 815-821,820A, XP002049672 ISSN: 0950-1991
- NAGY A ET AL: "Derivation of completely cell culture-derived mice from early-passage embryonic stem cells" PROCEEDINGS OF THE NATIONAL ACADEMY OF SCIENCES OF USA, NATIONAL ACADEMY OF SCIENCE. WASHINGTON, US, vol. 90, no. 18, 15 September 1993 (1993-09-15), pages 8424-8428, XP002182191 ISSN: 0027-8424
- CAMPBELL K H S ET AL: "SHEEP CLONED BY NUCLEAR TRANSFER FROM A CULTURED CELL LINE" NATURE, MACMILLAN JOURNALS LTD. LONDON, GB, vol. 380, no. 6569, 7 March 1996 (1996-03-07), pages 64-66, XP000605296 ISSN: 0028-0836
- WILMUT I ET AL: "VIABLE OFFSPRING DERIVED FROM FETAL AND ADULT MAMMALIAN CELLS" NATURE, MACMILLAN JOURNALS LTD. LONDON, GB, vol. 385, no. 6619, 27 February 1997 (1997-02-27), pages 810-813, XP002933216 ISSN: 0028-0836
- MODLINSKI J. A.: 'The Fate of Inner Cell Mass and Trophoectoderm Nuclei Transplanted to Fertilized Mouse Eggs' NATURE vol. 292, 23 July 1981, pages 342 - 343, XP002938872
- LUO J. ET AL.: 'Placental Abnormalities in Mouse Embryos Lacking the Orphan Nuclear Receptor ERR-beta' NATURE vol. 388, 21 August 1997, pages 778 - 782, XP002938871
- JAMES R. M. ET AL.: 'Restricted Distribution of Tetraploid Cell in Mouse Tetrapoloid (-) Diploid Chimeras' DEVELOPMENTAL BIOLOGY vol. 167, 1995, pages 213 - 226, XP002938870

## Description

### Background of the Invention

Nuclear transfer (or cloning) is a technique used to generate multiple identical embryos. It is performed as follows: unfertilized oocytes are collected. These oocytes can either be matured in vitro or in vivo. Oocytes at the appropriate stage of maturation can be enucleated. Following enucleation, oocytes can be fused to a donor cell or donor nuclei. The donor cell can be a somatic or non-somatic cell from an embryonic, fetal or adult mammal. The enucleated oocyte and the donor cell can either be simultaneously activated during fusion, or can be activated prior to or after fusion. For example, the oocyte can be activated after incubating the donor cell or nucleus with the enucleated oocyte for a period of time, e.g., up to 24 hours. Activation can then be achieved, for example, by chemical or physical means. The resulting embryo can then be transferred immediately or cultured in vitro and transferred at any time up to the blastocyst stage.

EPO 0 930 009 discloses the generation of chimeric animals from cells taken from a naturally formed embryo and an embryo reconstituted by nuclear transfer.

Luo et al (Nature 388:778-782) describe tetraploid rescue experiments whereby mutant ERR-β deficient embryos are rescued by associating the embryo with wild type tetraploid extra embryonic cells.

### Summary of the Invention

The present invention is based, in part, on the discovery that the development of an embryo into a later stage, e.g., into a post natal animal, can be optimized by providing cells which provide an extra-embryonic component to the developing organism. It has been discovered that the deleterious consequences of nuclear transfer manipulations can be reduced by transferring a reconstructed embryo in conjunction with a tetraploid aggregation. Although it is possible to obtain full-term pregnancies in several mammalian species, using reconstructed embryo techniques, a large number of these pregnancies are lost either early after implantation, or perinatally. Furthermore, offspring of cloned pregnancies often exhibit physiological defects and abnormalities. Such consequences may result from improper development of the extra-embryonic component of the cloned embryos. The use of extra-embryonic cells, e.g., a tetraploid aggregation, can be used to reduce defects of the extra-embryonic component of cloned embryos.

Accordingly, in one aspect, the invention features, use of an extra embryonic cell having a ploidy of greater than 2 in the preparation of a medicament for use in a method of promoting the development of a pre-natal non human animal, e.g., a embryo. The method includes providing the embryo with one or more extra-embryonic cells. While not wishing to be bound by theory, the inventors believe that the extra-embryonic cells contribute to or develop into placental or other non-embryonic tissue which promotes development. Thus, the method includes:
providing an embryo, e.g., a reconstructed embryo, in association with one or more extra-embryonic cells;
allowing the embryo to develop, in vivo or in vitro, to a later stage, e.g., allowing the embryo to complete a full term pregnancy to provide a post-natal non-human animal.

In a preferred embodiment the extra-embryonic cell is a cell other than a diploid cell, e.g., a cell with a ploidy of greater than 2, and in more preferred embodiments is a tetraploid cell. The extra-embryonic cell can be derived from a non-human embryo, e.g., an embryo in which the cells are fused to increase ploidy, e.g., a two cell stage embryo that has been tetraploidized.

In a preferred embodiment the embryo is a reconstructed embryo, e.g., it is the result of the combination of a genome of a first cell with a second functionally enucleated cell, e.g., an enucleated oocyte. By way of example, the reconstructed embryo can be formed by combining the genome of a cell, e.g., somatic cell (e.g., an embryonic, fetal or adult somatic cell), a stem cell or a stem cell-like cell (e.g., a cell obtained from an embryo up to the formation of an embryonic disc), an embryonic inner mass cell, with a recipient cell, e.g., functionally enucleated cell, e.g., an enucleated oocyte. The embryo can be cloned, genetically engineered, or both.

In a preferred embodiment, an unfertilized oocyte is functionally enucleated, e.g., enucleated. In a preferred embodiment, the oocyte is enucleated by physical or chemical means.

In a preferred embodiment, the genome of the cell, e.g., the genome of a somatic cell (e.g., a fibroblast) or non-somatic cell (e.g., a stem cell, stem cell-like cell, or a germ cell), is introduced into the oocyte by fusion. In a preferred embodiment, fusion occurs by electrical, chemical or physical means. In a preferred embodiment, the oocyte is activated prior to, simultaneously with, or after the introduction of the genome. In a preferred embodiment, the oocyte is: activated by electrofusion; activated by ionophore activation; activated by ethanol activation; in an activated stage, e.g., telophase. In a preferred embodiment, the reconstructed embryo is: implanted into recipient animal; implanted into a recipient animal following formation of the reconstructed embryo; cultured in vitro, e.g., cultured in vitro until two-cell stage, four cell stage, eight cell stage, sixteen cell stage, blastocyst stage.

In a preferred embodiment the method further includes allowing the non-human animal to develop to term and obtaining a useful substance, e.g., a protein, from the non-human animal or from a product, e.g., milk, of the non-human animal. In a preferred embodiment, the animal is a mammal, e.g., a goat, sheep, cow, pig, horse, rabbit, mouse, Ilama or camel. In a preferred embodiment, the mammal is a male or female mammal. In another preferred embodiment, the mammal is induced to lactate.

In another aspect the invention features, a method of promoting the development of a pre-natal non-human animal, e.g., an embryo. The method includes:
introducing a donor genome into a recipient enucleated cell to form reconstructed embryo; and
implanting the reconstructed embryo, in association with one or more extra-embryonic cells, into a recipient non-human mammal and allowing the embryo to develop into a later stage, e.g., a fetus or a postnatal non-human animal.

The reconstructed embryo is can be associated with the extra-embryonic cells at any time, but preferably prior to the time of implantation. Thus, the reconstructed embryo can be associated with the extra-embryonic cells prior to, at the time of, or after introduction of the donor genome. For example, the extra-embryonic cells can be associated with the recipient functionally enucleated cell, e.g., functionally enucleated oocyte, prior to or after introduction of the donor genome.

In a preferred embodiment the reconstructed embryo is allowed to develop to a later stage, e.g., it is allowed to complete a full term pregnancy to provide a post-natal non-human animal.

In a preferred embodiment the extra-embryonic cell is a cell other than a diploid cell, e.g., a cell with a ploidy of greater than 2, and in more preferred embodiments is a tetraploid cell. The extra-embryonic cell can be derived from a non-human embryo, e.g., a two cell stage embryo that has been tetraploidized. In a preferred embodiment the reconstructed embryo can be formed by combining the genome of a cell, e.g., somatic cell(e.g., an embryonic, fetal or adult somatic cell), a stem cell or a stem cell-like cell (e.g., a cell obtained from an embryo up to the formation of an embryonic disc), an embryonic inner mass cell, with a recipient cell, e.g., functionally enucleated cell, e.g., an enucleated oocyte. The embryo can be cloned, genetically engineered, or both.

In a preferred embodiment, an unfertilized oocyte is functionally enucleated, e.g., enucleated. In a preferred embodiment, the oocyte is enucleated by physical or chemical means.

In a preferred embodiment, the genome of the donor cell, e.g., the genome of a somatic cell or non-somatic cell (e.g., a stem cell, a stem cell-like cell, or a germ cell), is introduced into the oocyte by fusion. In a preferred embodiment, fusion occurs by electrical, chemical or physical means. In a preferred embodiment, the oocyte is activated prior to, simultaneously with, or after the introduction of the genome. In a preferred embodiment, the oocyte is: activated by electrofusion; activated by ionophore activation; activated by ethanol activation; in an activated stage, e.g., telophase.

In another aspect, the invention features use of an extra embryonic cell having a ploidy of greater than 2 in the preparation of a medicament for use in a method of inhibiting a defect in a cloned non-human mammal derived from a reconstructed embryo. The method includes combining at least one extra embryonic cell with a reconstructed embryo; transferring the embryo to a recipient mammal; and allowing the embryo to develop into a post-natal mammal.

In a preferred embodiment the extra-embryonic cell is a cell other than a diploid cell, e.g., a cell with a ploidy of greater than 2, and in more preferred embodiments is a tetraploid cell. The extra-embryonic cell can be derived from an embryo, e.g., an embryo in which the cells are fused to increase ploidy, e.g., a two cell stage embryo that has been tetraploidized

In a preferred embodiment, the reconstructed embryo can be formed by combining the genome of a cell, e.g., somatic cell (e.g., an embryonic, fetal or adult somatic cell), a stem cell or a stem cell-like cell (e.g., a cell obtained from an embryo up to the formation of an embryonic disc), an embryonic inner mass cell, with a recipient cell, e.g., functionally enucleated cell, e.g., an enucleated oocyte. The embryo can be cloned, genetically engineered, or both. In a preferred embodiment, an unfertilized oocyte is functionality enucleated, e.g., enucleated. In a preferred embodiment, the oocyte is enucleated by physical or chemical means. In a preferred embodiment, the genome of the cell, e.g., the genome of a somatic cell (e.g., a fibroblast) or non-somatic cell (e.g., a stem cell, stem cell-like cell, or a germ cell), is introduced into the oocyte by fusion. In a preferred embodiment, fusion occurs by electrical, chemical or physical means. In a preferred embodiment, the oocyte is activated prior to, simultaneously with, or after the introduction of the genome. In a preferred embodiment, the oocyte is: activated by electrofusion; activated by ionophore activation; activated by ethanol activation; in an activated stage, e.g., telophase. In a preferred embodiment, the reconstructed embryo is: implanted into recipient animal; implanted into a recipient animal following formation of the reconstructed embryo; cultured in vitro, e.g., cultured in vitro until two-cell stage, four cell stage, eight cell stage, sixteen cell stage, blastocyst stage.

In a preferred embodiment the method further includes allowing the non-human animal to develop to term and obtaining a useful substance, e.g., a protein, from the non-human animal or from a product, e.g., milk, of the non-human animal. In a preferred embodiment, the animal is a mammal, e.g., a goat, sheep, cow, pig, horse, rabbit, mouse, llama or camel. In a preferred embodiment, the mammal is a male or female mammal. In another preferred embodiment, the mammal is induced to lactate.

In another aspect, the invention features use of an extra embryonic cell having a ploidy of greater than 2 in the preparation of a medicament for use in a method of inhibiting spontaneous abortion of a reconstructed embryo by a recipient mammal. The method includes: combining at least one extra embryonic cell with a reconstructed embryo; transferring the embryo to a recipient mammal; and allowing the embryo to develop into a post-natal mammal.

In a preferred embodiment the extra-embryonic cell is a cell other than a diploid cell, e.g., a cell with a ploidy of greater than 2, and in more preferred embodiments is a tetraploid cell. The extra-embryonic cell can be derived from a non-human embryo, e.g., an embryo in which the cells are fused to increase ploidy, e.g., a two cell stage embryo that has been tetraploidized.

In a preferred embodiment, the reconstructed embryo can be formed by combining the genome of a cell, e.g., somatic cell (e.g., an embryonic, fetal or adult somatic cell), a stem cell or a stem cell-like cell (e.g., a cell obtained from an embryo up to the formation of an embryonic disc), an embryonic inner mass cell, with a recipient cell, e.g., functionally enucleated cell, e.g., an enucleated oocyte. The embryo can be cloned, genetically engineered, or both. In a preferred embodiment, an unfertilized oocyte is functionally enucleated, e.g., enucleated. In a preferred embodiment, the oocyte is enucleated by physical or chemical means. In a preferred embodiment, the genome of the cell, e.g., the genome of a somatic cell (e.g., a fibroblast) or non-somatic cell (e.g., a stem cell, stem cell-like cell, or a germ cell), is introduced into the oocyte by fusion. In a preferred embodiment, fusion occurs by electrical, chemical or physical means. In a preferred embodiment, the oocyte is activated prior to, simultaneously with, or after the introduction of the genome. In a preferred embodiment, the oocyte is: activated by electrofusion; activated by ionophore activation; activated by ethanol activation; in an activated stage, e.g., telophase. In a preferred embodiment, the reconstructed embryo is: implanted into recipient animal; implanted into a recipient animal following formation of the reconstructed embryo; cultured in vitro, e.g., cultured in vitro until two-cell stage, four cell stage, eight cell stage, sixteen cell stage, blastocyst stage.

In a preferred embodiment the method further includes obtaining a useful substance, e.g., a protein, from the post-natal animal or from a product, e.g., milk, of the post-natal animal. In a preferred embodiment, the animal is a mammal, e.g., a goat, sheep, cow, pig, horse, rabbit, mouse, llama or camel. In a preferred embodiment, the mammal is a male or female mammal. In another preferred embodiment, the mammal is induced to lactate.

In another aspect, the invention features, a method of making a cloned non-human mammal. The method includes: forming a reconstructed embryo having association with at least one extra embryonic cell having a ploidy of greater than 2; transferring the embryo into a recipient mammal; and allowing the embryo to develop into a post-natal mammal, thereby obtaining a cloned mammal.

In a preferred embodiment the extra-embryonic cell is a cell other than a diploid cell, e.g., a cell with a ploidy of greater than 2, and in more preferred embodiments is a tetraploid cell. The extra-embryonic cell can be derived from a non-human embryo, e.g., an embryo in which the cells are fused to increase ploidy, e.g., a two cell stage embryo that has been tetraploidized.

In a preferred embodiment, the reconstructed embryo can be formed by combining the genome of a cell, e.g., somatic cell (e.g., an embryonic, fetal or adult somatic cell), a stem cell or a stem cell-like cell (e.g., a cell obtained from an embryo up to the formation of an embryonic disc), an embryonic inner mass cell, with a recipient cell, e.g., functionally enucleated cell, e.g., an enucleated oocyte. The embryo can be cloned, genetically engineered, or both. In a preferred embodiment, an unfertilized oocyte is functionally enucleated, e.g., enucleated. In a preferred embodiment, the oocyte is enucleated by physical or chemical means. In a preferred embodiment, the genome of the cell, e.g., the genome of a somatic cell (e.g., a fibroblast) or non-somatic cell (e.g., a stem cell, stem cell-like cell, or a germ cell), is introduced into the oocyte by fusion. In a preferred embodiment, fusion occurs by electrical, chemical or physical means. In a preferred embodiment, the oocyte is activated prior to, simultaneously with, or after the introduction of the genome. In a preferred embodiment, the oocyte is: activated by electrofusion; activated by ionophore activation; activated by enthanol activation; in an activated stage, e.g., telophase. In a preferred embodiment, the reconstructed embryo is: implanted into recipient animal; implanted into a recipient animal following formation of the reconstructed embryo; culture in vitro, e.g., cultured in vitro until two-cell stage, four cell stage, eight cell stage, sixteen cell stage, blastocyst stage.

In a preferred embodiment the method further includes obtaining a useful substance, e.g., a protein, from the non-human mammal or from a product, e.g., milk, of the non-human mammal. In a preferred embodiment, the mammal is: a goat, sheep, cow, pig, horse, rabbit, mouse, llama or camel. In a preferred embodiment, the mammal is a male or a female mammal.

In another aspect, the invention features a method of making a transgenic non-human mammal. The method includes: introducing a genetically engineered genome of a mammalian donor cell into an oocyte to form a reconstructed embryo having association with at least one extra embryonic cell having a ploidy of greater than 2; transferring the embryo into a recipient mammal; and allowing the reconstructed embryo to develop into a post-natal mammal, thereby providing a transgenic mammal.

In a preferred embodiment the extra-embryonic cell is a cell other than a diploid cell, e.g., a cell with a ploidy of greater than 2, and in more preferred embodiments is a tetraploid cell. The extra-embryonic cell can be derived from a non-human embryo, e.g., an embryo in which the cells are fused to increase ploidy, e.g., a two cell stage embryo that has been tetraploidized.

In a preferred embodiment, the reconstructed embryo can be formed by combining the genome of a cell, e.g., somatic cell (e.g., an embryonic, fetal or adult somatic cell), a stem cell or a stem cell-like cell (e.g., a cell obtained from an embryo up to the formation of an embryonic disc), an embryonic inner mass cell, with a recipient cell, e.g., functionally enucleated cell, e.g., an enucleated oocyte.

In a preferred embodiment, an unfertilized oocyte is functionally enucleated, e.g., enucleated. In a preferred embodiment, the oocyte is enucleated by physical or chemical means.

In a preferred embodiment, the genome of the cell, e.g., the genome of a somatic cell (e.g., a fibroblast) or non-somatic cell (e.g., a stem cell, stem cell-like cell, or a germ cell), is introduced into the oocyte by fusion. In a preferred embodiment, fusion occurs by electrical, chemical or physical means. In a preferred embodiment, the oocyte is activated prior to, simultaneously with, or after the introduction of the genome. In a preferred embodiment, the oocyte is: activated by electrofusion; activated by ionophore activation; activated by ethanol activation; in an activated stage, e.g., telophase.

In a preferred embodiment, the reconstructed embryo is: implanted into recipient animal; implanted into a recipient animal following formation of the reconstructed embryo; cultured in vitro, e.g., cultured in vitro until two-cell stage, four cell stage, eight cell stage, sixteen cell stage, blastocyst stage.

In a preferred embodiment the method further includes obtaining a useful substance, e.g., a protein, from the non-human mammal or from a product, e.g., milk, of the non-human mammal. In a preferred embodiment, the mammal is: a goat, sheep, cow, pig, horse, rabbit, mouse, llama or camel. In a preferred embodiment, the mammal is a male or female mammal. In another preferred embodiment, the mammal is induced to lactate.

### Detailed Description of the Invention

### Donor Genomes

Donor genomes can be naturally occurring or genetically engineered. A donor genome can be derived from an embryonic, fetal or adult cell. The cell can be a somatic cell or a non-somatic cell, e.g., a cell which is not committed to a somatic cell lineage.

### Somatic Cells

Somatic cells can supply the genome for producing a reconstructed embryo. The term "somatic cell", as used herein, refers to a differentiated cell. The cell can be a somatic cell or a cell that is committed to a somatic cell lineage. Alternatively, a diploid stem cell that gives rise to a germ cell can be utilized in order to supply the genome for producing a reconstructed embryo.

The somatic cell can be from an animal or from a cell culture. If taken from an animal, the animal can be at any stage of development, e.g., an embryo, a fetus or an adult. Embryonic cells are preferred. Embryonic cells can include embryonic stem cells as well as embryonic cells committed to a somatic cell lineage. Such cells can be obtained from the endoderm, mesoderm or ectoderm of the embryo. Preferably, the embryonic cells are committed to somatic cell lineage. Embryonic cells committed to a somatic cell lineage refer to cells isolated on or after day 10 of embryogenesis. However, cells can be obtained prior to day ten of embryogenesis. If a cell line is used as a source of a chromosomal genome, primary cells are preferred. The term "primary cell line" as used herein includes primary cell lines as well as primary-derived cell lines.

Suitable somatic cells include fibroblasts (e.g., primary fibroblasts, e.g., embryonic primary fibroblasts), muscle cells (e.g., myocytes), cumulus cells, neural cells, and mammary cells. Other suitable cells include hepatocytes and pancreatic islets. Preferably, the somatic cell is an embryonic somatic cell, e.g., a cell isolated on or after day 10 of embryogenesis. The genome of the somatic cells can be the naturally occurring genome, e.g., for the production of cloned mammals, or the genome can be genetically altered to comprise a transgenic sequence, e.g., for the production of transgenic cloned mammals.

Somatic cells can be obtained by, for example, dissociation of tissue, e.g., by mechanical (e.g., chopping, mincing) or enzymatic means (e.g., trypsinization) to obtain a cell suspension and then by culturing the cells until a confluent monolayer is obtained. The somatic cells can then be harvested and prepared for cryopreservation, or maintained as a stock culture.

The somatic cell can be a quiescent or non-quiescent somatic cell. "Non-quiescent", as used herein, refers to a cell in mitotic cell cycle. The mitotic cell cycle has four distinct phases, G₁, S, G₂ and M. The beginning event in the cell cycle, called START, takes place during the G₁ phase. "START" as used herein refers to early G₁ stage of the cell cycle prior to the commitment of a cell to proceeding through the cell cycle. For example, 1, 2, 3, 4, 5, 6, 7, 8, 9, 10 up to 11 hours after a cell enters the G₁ stage, the cell is considered prior to START. The decision as to whether the cell will undergo another cell cycle is made at START. Once the cell has passed through START, it passes through the remainder of the G₁ phase (i.e., the pre-DNA synthesis stage). The S phase is the DNA synthesis stage, which is followed by the G₂ phase, the stage between synthesis and mitosis. Mitosis takes place during the M phase. If at START, the cell does not undergo another cell cycle, the cell becomes quiescent. In addition, a cell can be induced to exit the cell cycle and become quiescent. A "quiescent" cell, also referred to as a cell in G₀ phase, refers to a cell which is not in any of the four phases of the cell cycle. Preferably, the somatic cell is a cell in the G₀ phase or the G₁ phase of the mitotic cell cycle.

Using donor somatic cells at certain phases of the cell cycle, e.g., G₀ or G₁ phase, can allow for synchronization between the oocyte and the genome of the somatic cell. For example, reconstruction of an oocyte in metaphase II by introduction of a nucleus of a somatic cell in G₀ or G₁, e.g., by simultaneous activation and fusion, can mimic the events occurring during fertilization. By way of another example, an oocyte in telophase II fused, e.g., by simultaneous activation and fusion, with the genome of a somatic cell in G₁ prior to START, provides a synchronization of cell cycle between the oocyte and donor nuclei.

Methods of determining which phase of the cell cycle a cell is in are known. For example, as described below in the Examples, various markers are present at different stages of the cell cycle. Such markers can include cyclins D 1, 2, 3 and proliferating cell nuclear antigen (PCNA) for G₁, and BrDu to detect DNA synthetic activity. In addition, cells can be induced to enter the G₀ stage by culturing the cells on serum-deprived medium. These cells are also referred to herein as "serum starved cells". Alternatively, cells in G₀ stage can be induced to enter the cell cycle, i.e., at G₁ stage, by serum activation (also referred to as "non-serum starved cells").

### Genetically Engineered Somatic Cells:

### Transgenic Mammals

Methods for generating non-human transgenic mammals which can be used as a source of somatic cells for production of a reconstructed embryo are known in the art. Such methods can involve introducing DNA constructs into the germ line of a mammal to make a transgenic mammal. For example, one or several copies of the construct may be incorporated into the genome of a mammalian embryo by standard transgenic techniques.

Although goats are a preferred source of genetically engineered somatic cells, other non-human mammals can be used. Preferred non-human mammals are ruminants, e.g., cows, sheep, camels or goats. Goats of Swiss origin, e.g., the Alpine, Saanen and Toggenburg breed goats, are useful in the methods described herein. Additional examples of preferred non-human animals include oxen, horses, llamas, and pigs. The mammal used as the source of genetically engineered cells will depend on the transgenic mammal to be obtained by the methods of the invention as, by way of example, a goat genome should be introduced into a goat functionally enucleated oocyte.

Preferably, the somatic cells for use in the invention are obtained from a transgenic goat. Methods of producing transgenic goats are known in the art. For example, a transgene can be introduced into the germline of a goat by microinjection as described, for example, in Ebert et al. (1994) Biol/Technology 12:699, hereby incorporated by reference.

Other transgenic non-human animals to be used as a source of genetically engineered somatic cells can be produced by introducing a transgene into the germline of the non-human animal. Embryonal target cells at various developmental stages can be used to introduce transgenes. Different methods are used depending on the stage of development of the embryonal target cell. The specific line(s) of any animal used to practice this invention are selected for general good health, good embryo yields, good pronuclear visibility in the embryo, and good reproductive fitness. In addition, the haplotype is a significant factor.

### Transfected Cell Lines

Genetically engineered somatic cells can be obtained from a cell line into which a nucleic acid of interest, e.g., a nucleic acid which encodes a protein, has been introduced.

A construct can be introduced into a cell via conventional transformation or transfection techniques. As used herein, the terms "transfection" and "transformation" include a variety of techniques for introducing a transgenic sequence into a host cell, including calcium phosphate or calcium chloride co-precipitation, DEAE-dextrane-mediated transfection, lipofection, or electroporation. In addition, biological vectors, e.g., viral vectors can be used as described below. Suitable methods for transforming or transfecting host cells can be found in Sambrook et al., Molecular Cloning: A Laboratory Manuel, 2nd ed., Cold Spring Harbor Laboratory, (Cold Spring Harbor Laboratory Press, Cold Spring Harbor, NY, 1989), and other suitable laboratory manuals.

Two useful approaches are electroporation and lipofection. Brief examples of each are described below.

The DNA construct can be stably introduced into a donor somatic cell line by electroporation using the following protocol: somatic cells, e.g., fibroblasts, e.g., embryonic fibroblasts, are resuspended in PBS at about 4 x 10⁶ cells/ml. Fifty micorgrams of linearized DNA is added to the 0.5 ml cell suspension, and the suspension is placed in a 0.4 cm electrode gap cuvette (Biorad). Electroporation is performed using a Biorad Gene Pulser electroporator with a 330 volt pulse at 25 mA, 1000 microFarad and infinite resistance. If the DNA construct contains a Neomyocin resistance gene for selection, neomyocin resistant clones are selected following incubation with 350 microgram/ml of G418 (GibcoBRL) for 15 days.

The DNA construct can be stably introduced into a donor somatic cell line by lipofection using a protocol such as the following: about 2 x 10⁵ cells are plated into a 3.5 cmiameter well and transfected with 2 micrograms of linearized DNA using LipfectAMINE^{™} (GibcoBRL). Forty-eight hours after transfection, the cells are split 1:1000 and 1:5000 and, if the DNA construct contains a neomyosin resistance gene for selection, G418 is added to a final concentration of 0.35 mg/ml. Neomyocin resistant clones are isolated and expanded for cyropreservation as well as nuclear transfer.

### Constructs for Genetically Engineered Genomes

### Tissue-Specific Expression of Proteins

It is often desirable to express a protein, e.g., a heterologous protein, in a specific tissue or fluid, e.g., the milk, of a transgenic animal. The heterologous protein can be recovered from the tissue or fluid in which it is expressed. For example, it is often desirable to express the heterologous protein in milk. Methods for producing a heterologous protein under the control of a milk specific promoter are described below. In addition, other tissue-specific promoters, as well as, other regulatory elements, e.g., signal sequences and sequences which enhance secretion of non-secreted proteins, are described below.

### Milk Specific Promoters

Useful transcriptional promoters are those promoters that are preferentially activated in mammary epithelial cells, including promoters that control the genes encoding milk proteins such as caseins, beta lactoglobulin (Clark et al., (1989) Bio/Technology 7: 487-492), whey acid protein (Gordon et al. (1987) Bio/Technology 5: 1183-1187), and lactalbumin (Soulier et al., (1992) FEBS Letts. 297: 13). Casein promoters may be derived from the alpha, beta, gamma or kappa casein genes of any mammalian species; a preferred promoter is derived from the goat beta casein gene (DiTullio, (1992) Bio/Technology 10:74-77). Milk-specific protein promoter or the promoters that are specifically activated in mammary tissue can be derived from cDNA or genomic sequences. Preferably, they are genomic in origin. DNA sequence information is available for the mammary gland specific genes listed above, in at least one, and often in several organisms. See, e.g., Richards et al., J. Biol. Chem. 256, 526-532 (1981) (α-lactalbumin rat); Campbell et al., Nucleic Acids Res. 12, 8685-8697 (1984) (rat WAP); Jones et al., J. Biol. Chem. 260, 7042-7050 (1985) (rat β-casein); Yu-Lee & Rosen, J. Biol. Chem. 258, 10794-10804 (1983) (rat γ-casein); Hall, Biochem. J. 242, 735-742 (1987) (α-lactalbumin human); Stewart, Nucleic Acids Res. 12, 389 (1984) (bovine αs1 and κ casein cDNAs); Gorodetsky et al., Gene 66, 87-96 (1988) (bovine β casein); Alexander et al., Eur. J. Biochem. 178, 395-401 (1988) (bovine κ casein); Brignon et al., FEBS Lett. 188, 48-55 (1977) (bovine αS2 casein); Jamieson et al., Gene 61, 85-90 (1987), Ivanov et al., Biol. Chem. Hoppe-Seyler 369, 425-429 (1988), Alexander et al., Nucleic Acids Res. 17, 6739 (1989) (bovine β lactoglobulin); Vilotte et al., Biochimie 69, 609-620 (1987) (bovine α-lactalbumin). The structure and function of the various milk protein genes are reviewed by Mercier & Vilotte, J. Dairy Sci. 76, 3079-3098 (1993) (incorporated by reference in its entirety for all purposes). If additional flanking sequence are useful in optimizing expression of the heterologous protein, such sequences can be cloned using the existing sequences as probes. Mammary-gland specific regulatory sequences from different organisms can be obtained by screening libraries from such organisms using known cognate nucleotide sequences, or antibodies to cognate proteins as probes.

### Signal Sequences

Useful signal sequences are milk-specific signal sequences or other signal sequences which result in the secretion of eukaryotic or prokaryotic proteins. Preferably, the signal sequence is selected from milk-specific signal sequences, i.e., it is from a gene which encodes a product secreted into milk. Most preferably, the milk-specific signal sequence is related to the milk-specific promoter used in the construct, which are described below. The size of the signal sequence is not critical. All that is required is that the sequence be of a sufficient size to effect secretion of the desired recombinant protein, e.g., in the mammary tissue. For example, signal sequences from genes coding for caseins, e.g., alpha, beta, gamma or kappa caseins, beta lactoglobulin, whey acid protein, and lactalbumin can be used. A preferred signal sequence is the goat β-casein signal sequence.

Signal sequences from other secreted proteins, e.g., proteins secreted by kidney cells, pancreatic cells or liver cells, can also be used. Preferably, the signal sequence results in the secretion of proteins into, for example, urine or blood.

### Amino-Terminal Regions of Secreted Proteins

A non-secreted protein can also be modified in such a manner that it is secreted such as by inclusion in the protein to be secreted of all or part of the coding sequence of a protein which is normally secreted. Preferably the entire sequence of the protein which is normally secreted is not included in the sequence of the protein but rather only a sufficient portion of the amino terminal end of the protein which is normally secreted to result in secretion of the protein. For example, a protein which is not normally secreted is fused (usually at its amino terminal end) to an amino terminal portion of a protein which is normally secreted.

In one aspect, the protein which is normally secreted is a protein which is normally secreted in milk. Such proteins include proteins secreted by mammary epithelial cells, milk proteins such as caseins, beta lactoglobulin, whey acid protein, and lactalbumin. Casein proteins include alpha, beta, gamma or kappa casein genes of any mammalian species. A preferred protein is beta casein, e.g., goat beta casein. The sequence encoding the secreted protein can be derived from either cDNA or genomic sequences. Preferably, they are genomic in origin, and include one or more introns.

### Other Tissue-Specific Promoters

Other tissue-specific promoters which provide expression in a particular tissue can be used. Tissue specific promoters are promoters which are expressed more strongly in a particular tissue than in others. Tissue specific promoters are often expressed essentially exclusively in the specific tissue.

Tissue-specific promoters which can be used include: a neural-specific promoter, e.g., nestin, Wnt-1, Pax-1, Engrailed-1, Engrailed-2, Sonic hedgehog; a liver-specific promoter, e.g., albumin, alpha-1 antirypsin; a muscle-specific promoter, e.g., myogenin, actin, MyoD, myosin; an oocyte specific promoter, e.g., ZP1, ZP2, ZP3; a testes-specific promoter, e.g., protamin, fertilin, synaptonemal complex protein-1; a blood-specific promoter, e.g., globulin, GATA-1, porphobilinogen deaminase; a lung-specific promoter, e.g., surfactant protein C; a skin- or wool-specific promoter, e.g., keratin, elastin; endothelium-specific promoters, e.g., Tie-1, Tie-2; and a bone-specific promoter, e.g., BMP.

In addition, general promoters can be used for expression in several tissues. Examples of general promoters include β-actin, ROSA-21, PGK, FOS, c-myc, Jun-A, and Jun-B.

### Insulator Sequences

The DNA constructs used to make a transgenic reconstructed embryo can include at least one insulator sequence. The terms "insulator", "insulator sequence" and "insulator element" are used interchangeably herein. An insulator element is a control element which insulates the transcription of genes placed within its range of action but which does not perturb gene expression, either negatively or positively. Preferably, an insulator sequence is inserted on either side of the DNA sequence to be transcribed. For example, the insulator can be positioned about 200 bp to about 1 kb, 5' from the promoter, and at least about 1 kb to 5 kb from the promoter, at the 3' end of the gene of interest. The distance of the insulator sequence from the promoter and the 3' end of the gene of interest can be determined by those skilled in the art, depending on the relative sizes of the gene of interest, the promoter and the enhancer used in the construct. In addition, more than one insulator sequence can be positioned 5' from the promoter or at the 3' end of the transgene. For example, two or more insulator sequences can be positioned 5' from the promoter. The insulator or insulators at the 3' end of the transgene can be positioned at the 3' end of the gene of interest, or at the 3'end of a 3' regulatory sequence, e.g., a 3' untranslated region (UTR) or a 3' flanking sequence.

A preferred insulator is a DNA segment which encompasses the 5' end of the chicken β-globin locus and corresponds to the chicken 5' constitutive hypersensitive site as described in PCT Publication 94/23046, the contents of which is incorporated herein by reference.

### DNA Constructs

A cassette which encodes a heterologous protein can be assembled as a construct which includes a promoter for a specific tissue, e.g., for mammary epithelial cells, e.g., a casein promoter, e.g., a goat beta casein promoter, a milk-specific signal sequence, e.g., a casein signal sequence, e.g., a β-casein signal sequence, and a DNA encoding the heterologous protein.

The construct can also include a 3' untranslated region downstream of the DNA sequence coding for the non-secreted protein. Such regions can stabilize the RNA transcript of the expression system and thus increases the yield of desired protein from the expression system. Among the 3' untranslated regions useful in the constructs for use in the invention are sequences that provide a poly A signal. Such sequences may be derived, e.g., from the SV40 small t antigen, the casein 3' untranslated region or other 3' untranslated sequences well known in the art. In one aspect, the 3' untranslated region is derived from a milk specific protein. The length of the 3' untranslated region is not critical but the stabilizing effect of its poly A transcript appears important in stabilizing the RNA of the expression sequence.

Optionally, the construct can include a 5' untranslated region between the promoter and the DNA sequence encoding the signal sequence. Such untranslated regions can be from the same control region from which promoter is taken or can be from a different gene, e.g., they may be derived from other synthetic, semi-synthetic or natural sources. Again their specific length is not critical, however, they appear to be useful in improving the level of expression.

The construct can also include about 10%, 20%, 30%, or more of the N-terminal coding region of a gene preferentially expressed in mammary epithelial cells. For example, the N-terminal coding region can correspond to the promoter used, e.g., a goat β-casein N-terminal coding region.

The construct can be prepared using methods known in the art. The construct can be prepared as part of a larger plasmid. Such preparation allows the cloning and selection of the correct constructions in an efficient manner. The construct can be located between convenient restriction sites on the plasmid so that they can be easily isolated from the remaining plasmid sequences for incorporation into the desired mammal.

### Heterologous Proteins

Transgenic sequences encoding heterologous proteins can be introduced into the germline of a non-human mammal or can be transfected into a cell line to provide a source of genetically engineered somatic cells as described above.

The protein can be a complex or multimeric protein, e.g., a homo- or heteromultimer, e.g., proteins which naturally occur as homo- or heteromultimers, e.g., homo- or hetero- dimers, trimers or tetramers. The protein can be a protein which is processed by removal, e.g., cleavage, of N-terminus, C-terminus or internal fragments. Even complex proteins can be expressed in active form. Protein encoding sequences which can be introduced into the genome of mammal, e.g., goats, include glycoproteins, neuropeptides, immunoglobulins, enzymes, peptides and hormones. The protein may be a naturally occurring protein or a recombinant protein, e.g., a fragment, fusion protein, e.g., an immunoglogulin fusion protein, or mutien. It may be human or non-human in origin. The heterologous protein may be a potential therapeutic or pharmaceutical agent such as, but not limited to: alpha-1 proteinase inhibitor, alpha-1 antitrypsine, alkaline phosphatase, angiogenin, antithrombin III, any of the blood clotting factors including Factor VIII, Factor IX, and Factor X, chitinase, decorin, erythropoietin, extracellular superoxide dismutase, fibrinogen, glucocerebrosidase, glutamate decarboxylase, human growth factor, human serum albumin, immunoglobulin, insulin, myelin basic protein, platelet derived growth factor, proinsulin, prolactin, soluble CD4 or a component or complex thereof, lactoferrin, lactoglobulin, lysozyme, lactalbumin, tissue plasminogen activator or a variant thereof.

Immunoglobulins are particularly preferred heterologous protiens. Examples of immunoglobulins include IgA, IgG, IgE, IgM, chimeric antibodies, humanized antibodies, recombinant antibodies, single chain antibodies and antibody-protein fusions.

Nucleotide sequence information is available for several of the genes encoding the heterologous proteins listed above, in at least one, and often in several organisms. *See e.g.,* Long et al. (1984) Biochem. 23(21):4828-4837 (aplha-1 antitrypsin); Mitchell et al. (1986) Prot. Natl. Acad. Sci USA 83:7182-7186 (alkaline phosphatase); Schneider et al. (1988) EMBO J. 7(13):4151-4156 (angiogenin); Bock et al. (1988) Biochem. 27(16):6171-6178 (antithrombin III); Olds et al. (1991) Br. J. Haematol. 78(3):408-413 (antithrombin III); Lin et al. (1985) Proc. Natl. Acad. Sci. USA 82(22):7580-7584 (erythropoeitin); U.S. Patent No. 5,614,184 (erythropoietin); Horowitz et al. (1989) Genomics 4(1):87-96 (glucocerebrosidase); Kelly et al. (1992) Ann. Hum. Genet. 56(3):255-265 (glutamte decarboxylase); U.S. Patent No. 5,707,828 (human serum albumin); U.S. Patent No. 5,652,352 (human serum albumin); Lawn et al. (1981) Nucleic Acid Res. 9(22):6103-6114 (human serum albumin); Kamholz et al. (1986) Prot. Natl. Acad Sci. USA 83(13):4962-4966 (myelin basic protein); Hiraoka et al. (1991) Mol. Cell Endocrinol. 75(1):71-80 (prolactin); U.S. Patent No. 5,571,896 (lactoferrin); Pennica et al. (1983) Nature 301(5897):214-221 (tissue plasminogen activator); Sarafanov et al. (1995) Mol. Biol. 29:161-165, the contents of which are incorporated herein by reference.

### Recipient Cells

Any functionally enucleated cell which can support development into a later stage can be used. Oocytes are particularly preferred.

### Oocytes

Oocytes which can be used to make a reconstructed embryo include oocytes in metaphase II stage, e.g., oocytes arrested in metaphase II, and telophase II. Oocytes in metaphase II contain one polar body, whereas oocytes in telophase can be identified based on the presence of a protrusion of the plasma membrane from the second polar body up to the formation of a second polar body. In addition, oocytes in metaphase II can be distinguished from oocytes in telophase II based on biochemical and/or developmental distinctions. For example, oocytes in metaphase II can be in an arrested state, whereas oocytes in telophase are in an activated state. Preferably, the oocyte is a caprine oocyte. Other preferred sources of oocytes include sheep, cows, pigs, llama, camel, rabbit, and mice.

Occytes can be obtained at various times during a mammal's reproductive cycle. For example, at given times during the reproductive cycle, a significant percentage of the oocytes, e.g., about 55%, 60%, 65%, 70%, 75%, 80% or more, are oocytes in telophase. In addition, oocytes at various stages of the cell cycle can be obtained and then induced *in vitro* to enter a particular stage of meiosis. For example, oocytes cultured on serum-starved medium become arrested in metaphase. In addition, arrested oocytes can be induced to enter telophase by serum activation. Thus, oocytes in telophase can be easily obtained for use in the invention.

Oocytes can be matured *in vitro* before they are used to form a reconstructed embryo. This process usually requires collecting immature oocytes from mammalian ovaries, and maturing the oocyte in a medium prior to enucleation until the oocyte reaches the desired meiotic stage, e.g., metaphase or telophase. In addition, oocytes that have been matured *in vivo* can be used to form a reconstructed embryo.

Oocytes can be collected from a female mammal during superovulation. Briefly, oocytes can be recovered surgically by flushing the oocytes from the oviduct of the female donor. Methods of inducing superovulation and the collection of oocytes are known.

Preferably, the mitotic stage of the oocyte, e.g., metaphase II or telophase II, correlates to the stage of the cell cycle of the donor somatic cell. The correlation between the meiotic stage of the oocyte and the mitotic stage of the cell cycle of the donor somatic cell is referred to herein as "synchronization". For example, reconstruction of an oocyte in metaphase II by introduction of a nucleus of a somatic cell in G₀ or G₁, e.g., by simultaneous activation and fusion, can mimic the events occurring during fertilization. By way of another example, an oocyte in telophase fused, e.g., by simultaneous activation and fusion, with the genome of a somatic cell in G₁ prior to START, provides a synchronization between the oocyte and the donor nuclei.

### Functional Enucleation

The donor oocyte should be functionally enucleated such that the endogenous genome of the oocyte is incapable of functioning, e.g., replicating or synthesizing DNA. Methods of functionally enucleating an oocyte include: removing the genome from the oocyte (i.e., enucleation); inactivating DNA within the oocyte, e.g., by irradiation (e.g., by X-ray irradiation, or laser irradiation); chemical inactivation, or the like.

### Enucleation

One method of rendering the genome of an oocyte incapable of functioning is to remove the genome from the oocyte (i.e., enucleation). A micropipette or needle can be inserted into the zona pellicuda in order to remove nuclear material from an oocyte. For example, metaphase II stage oocytes which have one polar body can be enucleated with a micropipette by aspirating the first polar body and adjacent cytoplasm surrounding the polar body, e.g., approximately 20%, 30%, 40%, 50%, 60% of the cytoplasm, which presumably contains the metaphase plate. Telphase stage oocytes which have two polar bodies can be enucleated with a micropipette or needle by removing the second polar body and surrounding cytoplasm, e.g., approximately 5%, 10%, 20%, 30%, 40%, 50%, 60% of cytoplasm. Specifically, oocytes in telophase stage can be enucleated at any point from the presence of a protrusion in the plasma membrane from the second polar body up to the formation of the second polar body. Thus, as used herein, oocytes which demonstrate a protrusion in the plasma membrane, usually with a spindle abutted to it, up to extrusion of the second polar body are considered to be oocytes in telophase. Alternatively, oocytes which have one clear and distinct polar body with no evidence of protrusion are considered to be oocytes in metaphase.

### Irradiation

The oocyte can be functionally enucleated by inactivating the endogenous DNA of the oocyte using irradiation. Methods of using irradiation are known in the art and described, for example, in Bradshaw et al. (1995) Molecul. Reprod Dev. 41:503-512, the contents of which is incorporated herein by reference.

### Chemical Inactivation

The oocyte can be functionally enucleated by chemically inactivating the endogenous DNA of the oocyte. Methods of chemically inactivating the DNA are known in the art. For example, chemical inactivation can be performed using the etopsoide-cycloheximide method as described in Fulkaj and Moore (1993) Molecul. Reprod. Dev. 34:427-430, the content of which are incorporated herein by reference.

### Introduction of a Functional Chromosomal Genome into an Oocyte

Methods described herein can include the introduction of a functional non-human chromosomal genome into an oocyte, e.g., a functionally enucleated oocyte, e.g., an enucleated oocyte, to form a reconstructed embryo. The functional chromosomal genome directs the development of a cloned or transgenic animal which arises from the reconstructed embryo. Methods which result in the transfer of an essentially intact chromosomal genome to the oocyte can be used. Examples include fusion of a cell which contains the functional chromosomal genome with the oocyte and nuclear injection, i.e., direct transfer of the nucleus into the oocyte.

### Fusion

Fusion of the somatic cell with an oocyte can be performed by, for example, electrofusion, viral fusion, biochemical reagent fusion (e.g., HA protein), or chemical fusion (e.g., with polyethylene glycol (PEG) or ethanol).

Fusion of the somatic cell with the oocyte and activation can be performed simultaneously. For example, the nucleus of the somatic cell can be deposited within the zona pelliduca which contains the oocyte. The steps of fusing the nucleus with the oocyte and activation can then be performed simultaneously by, for example, applying an electric field. Fusion of the somatic cell with an oocyte can also be performed prior to or after activation of the oocyte.

### Activation of a Recombinant Embryo

Activation refers to the beginning of embryonic development, e.g., replication and DNA synthesis. Activation can be induced by, for example, electric shock (e.g., in electrofusion), the use of ionophores, ethanol activation, or the oocyte can be obtained during a stage in which it is naturally activated, e.g., an oocyte in telophase.

### Electrofusion

A reconstructed embryo can be activated using electric shock, i.e., electrofusion. The use of electrofusion allows for the fusion of the somatic cell with the oocyte and activation to be performed simultaneously.

Chambers, such as the BTX 200 Embryomanipulation System, for carrying out electrofusion are commercially available from, for example, BTX, San Diego.

### Ionophores

In addition, the reconstructed embryo can be activated by ionophore activation. Using an ionophore, e.g., a calcium ionophore, the calcium concentration across the membrane of the reconstructed embryo is changed. As the free calcium concentration in the cell increases, there is a decrease in phosphorylation of intracellular proteins and the oocyte is activated. Such methods of activation are described, for example, in U.S. Patent Number 5,496,720, the contents of which are incorporated by reference.

### Ethanol Activation

Prior to enucleation, an oocyte, e.g., an oocyte in metaphase II, can be activated with ethanol according to the ethanol activation treatment as described in Presicce and Yang (1994) Mol. Reprod. Dev. 37:61-68, and Bordignon and Smith (1998) Mol. Reprod. Dev. 49:29-36, the contents of which are incorporated herein by reference.

### Ooctyes in Telophase

Oocytes in telophase are generally already activated. Thus, these cells often naturally exhibit a decrease in calcium concentration which prevents fertilization and allows the embryo to develop.

### Extra-Embryonic Cells

In a preferred embodiment, the extra-embryonic cell is one which is exogenously added to the embryo. An extra-embryonic cell refers to a cell which does not develop into the embryo (i.e., the embryo proper) but rather a support tissue such as the placenta. For example, cells of the trophectoderm are extra embryonic cells which contribute to placental development. Preferably, the extra-embryonic cell is a cell which has a ploidy of greater than two, e.g., a tetraploid cell. The extra-embryonic cell can be derived from a non-human embryo or oocyte, e.g., a cell having a ploidy of greater than 2 derived from an embryo or oocyte.

### Tetraploidization

A tetraploid embryo can be generated using a non-human pre-implantation embryos, preferably but not necessarily, at the 2-cell stage, by submitting these embryo to a tetraploidization procedure. The terms "tetraploid embryo" refers to an activated oocyte which has been subjected to a tetraploidization procedure. Tetraploidization can be achieved by physical, chemical or electrical means. Tetraploidization is used to generate embryonic cells that have twice the genetic complement of a normal cell. For example, a 2-cell stage embryo can be induced, e.g., using electrical fusion, to recreate a one-cell embryo in which the genetic complement is 4N rather than 2N, as observed in unmanipulated 2-cell stage embryos.

Electrofusion can be used to generate a tetraploid embryo. For example, an embryo, e.g., a 2-cell stage embryo, can be exposed to electrical field pulses which cause degradation of the cell membrane and cell fusion to produce a tetraploid embryo, e.g., to produce a one-cell tetraploid embryo from a 2-cell stage embryo. Methods of tetraploidization by electrical means can be performed as described, for example, in James et al. (1995) Dev. Biol. 167:213-226; Prather et al. (1996) Mol. Reprod. & Develop. 45:38-42; Duncan et al. (1997) Development 124:279-287, which are incorporated herein by reference.

Tetraploid embryos can also be generated using chemical means. For example, cytochalasin B (Sigma, St. Louis, MO) can be used to generate a tetraploid embryo. Such methods are described, for example, in Koizumi et al. (1995) Exp. Anim. 44(2):105-109; Koizumi et al. (1996) Exp. Anim. 45(2):179-181, the contents of which are incorporated by reference.

After the tetraploidization procedure, the tetraploid embryo can be cultured to at least the one-cell stage up to the blastocyst stage. The tetraploid embryo can then be treated with chemical, biological or physical means to remove the zona pellucida, and the tetraploid cells are aggregated, referred to herein as a "tetraploid aggregation", to the cell(s) of the reconstructed embryo. The reconstructed embryo can be at any stage from the one-cell stage up to the blastocyst stage. The tetraploid embryo to which the cloned embryo is aggregated (or added) can also be at any stage from the one-cell stage to the blastocyst stage. The tetraploid embryo and the cloned embryo do not need to be at the same stage of development. Such procedures can also be performed by aggregating tetraploid embryonic stem cells to a cloned embryo at any stage of development.

The resulting tetraploid aggregate/clone embryo, also referred to herein as a "couplet", can then be cultured in vitro up to the blastocyst stage or can be transferred immediately to a pseudopregnant recipient. The resulting couplet can also be cultured in vivo in an intermediate recipient prior to transfer into a pseudopregnant recipient.

### Transfer of Reconstructed Embryos

A reconstructed embryo can be transferred to a recipient doe and allowed to develop into a cloned or transgenic mammal. For example, the reconstructed embryo can be transferred via the fimbria into the oviductal lumen of each recipient doe as described below in the Examples. In addition, methods of transferring an embryo to a recipient mammal are known in the art and described, for example, in Ebert et al. (1994) Biol/Technology 12:699.

The reconstructed embryo can be maintained in a culture until at least first cleavage (2-cell stage) up to blastocyst stage, preferably the embryos are transferred at 2-cell or 4 cell-stage. Various culture media for embryo development are known in the art. For example, the reconstructed embryo can be co-cultured with oviductal epithelial cell monolayer derived from the type of mammal to be provided by the invention.

### Purification of Proteins from Milk

The transgenic protein can be produced in milk at relatively high concentrations and in large volumes, providing continuous high level output of normally processed peptide that is easily harvested from a renewable resource. There are several different methods known in the art for isolation of proteins form milk.

Milk proteins usually are isolated by a combination of processes. Raw milk first is fractionated to remove fats, for example, by skimming, centrifugation, sedimentation (H.E. Swaisgood, Developments in Dairy Chemistry, I: Chemistry of Milk Protein, Applied Science Publishers, NY, 1982), acid precipitation (U.S. Patent No. 4,644,056) or enzymatic coagulation with rennin or chymotrypsin (Swaisgood, ibid.). Next, the major milk proteins may be fractionated into either a clear solution or a bulk precipitate from which the specific protein of interest may be readily purified.

French Patent No. 2487642 describes the isolation of milk proteins from skim milk or whey by membrane ultrafiltration in combination with exclusion chromatography or ion exchange chromatography. Whey is first produced by removing the casein by coagulation with rennet or lactic acid. U.S. Patent No. 4,485,040 describes the isolation of an alpha-lactoglobulin-enriched product in the retentate from whey by two sequential ultrafiltration steps. U.S. Patent No. 4,644,056 provides a method for purifying immunoglobulin from milk or colostrum by acid precipitation at pH 4.0-5.5, and sequential cross-flow filtration first on a membrane with 0.1 - 1.2 micrometer pore size to clarify the product pool and then on a membrane with a separation limit of 5 - 80 kd to concentrate it.

Similarly, U.S. Patent No. 4,897,465 teaches the concentration and enrichment of a protein such as immunoglobulin from blood serum, egg yolks or whey by sequential ultrafiltration on metallic oxide membranes with a pH shift. Filtration is carried out first at a pH below the isoelectric point (pI) of the selected protein to remove bulk contaminants from the protein retentate, and next at a pH above the pI of the selected protein to retain impurities and pass the selected protein to the permeate. A different filtration concentration method is taught by European Patent No. EP 467 482 B1 in which defatted skim milk is reduced to pH 3-4, below the pI of the milk proteins, to solubilize both casein and whey proteins. Three successive rounds of ultrafiltration or diafiltration then concentrate the proteins to form a retentate containing 15-20% solids of which 90% is protein. Alternatively, British Patent Application No. 2179947 discloses the isolation of lactoferrin from whey by ultrafiltration to concentrate the sample, followed by weak cation exchange chromatography at approximately a neutral pH. No measure of purity is reported. In PCT Publication No. WO 95/22258, a protein such as -lactoferrin is recovered from milk that has been adjusted to high ionic strength by the addition of concentrated salt, followed by cation exchange chromatography.

In all of these methods, milk or a fraction thereof is first treated to remove fats, lipids, and other particulate matter that would foul filtration membranes or chromatography media. The initial fractions thus produced may consist of casein, whey, or total milk protein, from which the protein of interest is then isolated.

PCT Patent Publication No. WO 94/19935 discloses a method of isolating a biologically active protein from whole milk by stabilizing the solubility of total milk proteins with a positively charged agent such as arginine, imidazole or Bis-Tris. This treatment forms a clarified solution from which the protein may be isolated, e.g., by filtration through membranes that otherwise would become clogged by precipitated proteins.

USSN 08/648,235 discloses a method for isolating a soluble milk component, such as a peptide, in its biologically active form from whole milk or a milk fraction by tangential flow filtration. Unlike previous isolation methods, this eliminates the need for a first fractionation of whole milk to remove fat and casein micelles, thereby simplifying the process and avoiding losses of recovery and bioactivity. This method may be used in combination with additional purification steps to further remove contaminants and purify the product, e.g., protein, of interest.

## Claims

1. Use of an extra-embryonic cell having a ploidy of greater than 2 in the preparation of a medicament for use in a method of promoting the development of a pre-natal non-human mammal, the method comprising:
forming a reconstructed embryo from non-human nucleic and non-human recipient cells in association with at least one extra embryonic cell;
transferring the reconstructed embryo into a non-human recipient mammal; and
allowing the embryo to develop, and allowing the extra-embryonic cell to develop into non-embryonic tissue, to thereby promote development of the mammal, provided the extra-embryonic cell is not derived from a human embryo.

2. The use of claim 1, wherein the extra embryonic cell is a tetraploid cell.

3. The use of claim 1, wherein the extra-embryonic cell is derived from a non-human embryo in which the cells are fused to increase ploidy.

4. The use of claim 1, wherein the reconstructed embryo is a genetically engineered reconstructed embryo.

5. The use of claim 1, wherein the reconstructed embryo is transferred at about the two-cell stage to the blastocyst stage.

6. Use of an extra-embryonic cell having a ploidy of greater than 2 in the preparation of a medicament for use in a method of decreasing the rate of spontaneous abortion of a reconstructed embryo by a recipient non-human mammal, the method comprising:
combining at least one extra embryonic cell with an embryo reconstructed from non-human nucleic and non-human recipient cells;
transferring the embryo to a recipient mammal; and
allowing the embryo to develop into a post-natal mammal, and the extra-embryonic cell to develop into non-embryonic tissue, thereby decreasing the rate of spontaneous abortion of the reconstructed embryo by a recipient mammal, provided the extra-embryonic cell is not derived from a human embryo.

7. The use of claim 6, wherein the extra embryonic cell is a tetraploid cell.

8. The use of claim 6, wherein the extra-embryonic cell is derived from a non-human embryo in which the cells are fused to increase ploidy.

9. The use of claim 6, wherein the reconstructed embryo is a gentically engineered reconstructed embryo.

10. The use of claim 6, wherein the reconstructed embryo is transferred at about the two-cell stage to the blastocyst stage.

11. Use of an extra-embryonic cell having a ploidy of greater than 2 in the preparation of a medicament for use in a method of decreasing the number of defects in a cloned non-human mammal derived from an embryo reconstructed from non-human nucleic and non-human recipient cells the method comprising:
combining at least one extra embryonic cell with a reconstructed embryo;
transferring the embryo to a non-human recipient mammal; and
allowing the embryo to develop into a post-natal mammal, and the extra-embryonic cell to develop into non-embryonic tissue, thereby decreasing the number of defects in a cloned non-human mammal derived from a reconstructed embryo, provided the extra-embryonic cell is not derived from a human embryo.

12. The use of claim 11, wherein the extra embryonic cell is a tetraploid cell.

13. The use of claim 11, wherein the extra-embryonic cell is derived from a non-human embryo in which the cells are fused to increase ploidy.

14. The use of claim 11, wherein the reconstructed embryo is a genetically engineered reconstructed embryo.

15. The use of claim 11, wherein the reconstructed embryo is transferred at about the two-cell stage to the blastocyst stage.

16. A method of making a cloned non-human mammal, the method comprising:
forming a reconstructed embryo from non-human nucleic and non-human recipient cells having association with at least one extra embryonic cell having a ploidy of greater than 2;
transferring the embryo into a recipient non-human mammal; and
allowing the embryo to develop into a post-natal mammal, and allowing the extra embryonic cell to develop into non-embryonic tissue, thereby obtaining a cloned non-human mammal, provided the extra-embryonic cell is not derived from a human embryo.

17. A method of making a transgenic non-human mammal, the method comprising:
introducing a genetically engineered genome of a non-human mammalian donor cell into a non-human oocyte to form a reconstructed embryo having association with at least one extra embryonic cell having a ploidy of greater than 2; transferring the embryo into a recipient non-human mammal; and allowing the embryo to develop into a post-natal mammal, and allowing the extra embryonic cell to develop into non-embryonic tissue, thereby providing a transgenic non-human mammal, provided the extra-embryonic cell is not derived from a human embryo.

## Patentansprüche

1. Verwendung einer extraembryonalen Zelle mit einer Ploidie von mehr als 2 bei der Herstellung eines Medikaments zur Verwendung in einem Verfahren zur Förderung der Entwicklung eines pränatalen nicht-humanen Säugers, wobei das Verfahren umfasst:
Erzeugen eines aus nicht-humane Zellkerne und nicht-humanen Empfängerzellen rekonstruierten Embryos in Verbindung mit mindestens einer extraembryonalen Zelle;
Überführen des rekonstruierten Embryos in einen nicht-humanen Empfängersäuger; und
Entwickelnlassen des Embryos und Entwickelnlassen der extraembryonalen Zelle zu nicht-embryonalem Gewebe, um **dadurch** die Entwicklung des Säugers zu fördern, mit der Maßgabe, daß die extraembryonale Zelle nicht von einem humanen Embryo abgeleitet ist.

2. Verwendung nach Anspruch 1, wobei die extraembryonale Zelle eine tetraploide Zelle ist.

3. Verwendung nach Anspruch 1, wobei die extraembryonale Zelle von einem nicht-humanen Embryo abgeleitet ist, bei dem die Zellen verschmolzen sind, um die Ploidie zu erhöhen.

4. Verwendung nach Anspruch 1, wobei der rekonstruierte Embryo ein genetisch veränderter rekonstruierter Embryo ist.

5. Verwendung nach Anspruch 1, wobei der rekonstruierte Embryo in etwa dem Zweizellenstadium in das Blastozystenstadium überführt wird.

6. Verwendung einer extraembryonalen Zelle mit einer Ploidie von mehr als 2 bei der Herstellung eines Medikaments zur Verwendung in einem Verfahren zur Verminderung der Rate von spontanem Abort eines rekonstruierten Embryos durch einen nicht-humanen Empfängersäuger, wobei das Verfahren umfasst:
Vereinigen mindestens einer extraembryonalen Zelle mit einem aus nicht-humane Zellkerne und nicht-humanen Empfängerzellen rekonstruierten nicht-humanen Embryo;
Überführen des Embryos in einen Empfängersäuger; und
Entwickelnlassen des Embryos zu einem postnatalen Säuger und Entwickelnlassen der extraembryonalen Zelle zu nicht-embryonalem Gewebe, **dadurch** Vermindern der Rate von spontanem Abort des rekonstruierten Embryos durch einen Empfängersäuger, mit der Maßgabe, daß die extraembryonale Zelle nicht von einem humanen Embryo abgeleitet ist.

7. Verwendung nach Anspruch 6, wobei die extraembryonale Zelle eine tetraploide Zelle ist.

8. Verwendung nach Anspruch 6, wobei die extraembryonale Zelle von einem nicht-humanen Embryo abgeleitet ist, bei dem die Zellen verschmolzen sind, um die Ploidie zu erhöhen.

9. Verwendung nach Anspruch 6, wobei der rekonstruierte Embryo ein genetisch veränderter rekonstruierter Embryo ist.

10. Verwendung nach Anspruch 6, wobei der rekonstruierte Embryo in etwa dem Zweizellenstadium in das Blastozystenstadium überführt wird.

11. Verwendung einer extraembryonalen Zelle mit einer Ploidie von mehr als 2 bei der Herstellung eines Medikaments zur Verwendung in einem Verfahren zur Verminderung der Anzahl von Defekten in einem geklonten nicht-humanen Säuger, abgeleitet von einem rekonstruierten Embryo, wobei das Verfahren umfasst:
Vereinigen mindestens einer extraembryonalen Zelle mit einem aus nicht-humane Zellkerne und nicht-humanen Empfängerzellen rekonstruierten Embryo;
Überführen des Embryos in einen nicht-humanen Empfängersäuger; und
Entwickelnlassen des Embryos zu einem postnatalen Säuger und Entwickelnlassen der extraembryonalen Zelle zu nicht-embryonalem Gewebe, **dadurch** Vermindern der Anzahl von Defekten in einem geklonten nicht-humanen Säuger, abgeleitet von einem rekonstruierten Embryo, mit der Maßgabe, daß die extraembryonale Zelle nicht von einem humanen Embryo abgeleitet ist.

12. Verwendung nach Anspruch 11, wobei die extraembryonale Zelle eine tetraploide Zelle ist.

13. Verwendung nach Anspruch 11, wobei die extraembryonale Zelle von einem nicht-humanen Embryo abgeleitet ist, bei dem die Zellen verschmolzen sind, um die Ploidie zu erhöhen.

14. Verwendung nach Anspruch 11, wobei der rekonstruierte Embryo ein genetisch veränderter rekonstruierter Embryo ist.

15. Verwendung nach Anspruch 11, wobei der rekonstruierte Embryo in etwa dem Zweizellenstadium in das Blastozystenstadium überführt wird.

16. Verfahren zum Herstellen eines geklonten nicht-humanen Säugers, wobei das Verfahren umfasst:
Erzeugen eines aus nicht-humane Zellkerne und nicht-humanen Empfängerzellen rekonstruierten Embryos mit Verbindung mit mindestens einer extraembryonalen Zelle mit einer Ploidie von mehr als 2;
Überführen des Embryos in einen nicht-humanen Empfängersäuger; und
Entwickelnlassen des Embryos zu einem postnatalen Säuger und Entwickelnlassen der extraembryonalen Zelle zu nicht-embryonalem Gewebe, **dadurch** Erhalten eines geklonten nicht-humanen Säugers, mit der Maßgabe, daß die extraembryonale Zelle nicht von einem humanen Embryo abgeleitet ist.

17. Verfahren zum Herstellen eines transgenen nicht-humanen Säugers, wobei das Verfahren umfasst:
Einführen eines genetisch veränderten Genoms einer nichthumanen Säugerspenderzelle in einen nicht-humanen Oozyten, um einen rekonstruierten Embryo mit Verbindung mit mindestens einer extraembryonalen Zelle mit einer Ploidie von mehr als 2 zu erzeugen; Überführen des Embryos in einen nicht-humanen Empfängersäuger; und Entwickelnlassen des Embryos zu einem postnatalen Säuger und Entwickelnlassen der extraembryonalen Zelle zu nicht-embryonalem Gewebe, **dadurch** Bereitstellen eines transgenen nicht-humanen Säugers, mit der Maßgabe, daß die extraembryonale Zelle nicht von einem humanen Embryo abgeleitet ist.

## Revendications

1. Utilisation d'une cellule extra-embryonnaire ayant une ploïdie supérieure à 2 pour la préparation d'un médicament pour une utilisation dans un procédé pour favoriser le développement d'un mammifère non humain pré-natal, le procédé comprenant:
la formation d'un embryon reconstruit à partir de noyaux non humains et de cellules receveuses non humaines conjointement avec au moins une cellule extra-embryonnaire;
le transfert de l'embryon reconstruit à un mammifère receveur non humain; et
le fait de permettre à l'embryon de se développer, et de permettre à la cellule extra-embryonnaire de se développer en un tissu non embryonnaire, pour favoriser de cette manière le développement du mammifère, à condition que la cellule extra-embryonnaire ne soit pas issue d'un embryon humain.

2. Utilisation selon la revendication 1, dans laquelle la cellule extra-embryonnaire est une cellule tétraploïde.

3. Utilisation selon la revendication 1, dans laquelle la cellule extra-embryonnaire est issue d'un embryon non humain dont les cellules sont fusionnées pour augmenter la ploïdie.

4. Utilisation selon la revendication 1, dans laquelle l'embryon reconstruit est un embryon reconstruit modifié génétiquement.

5. Utilisation selon la revendication 1, dans laquelle l'embryon reconstruit est transféré environ au stade bicellulaire jusqu'au stade de blastocyste.

6. Utilisation d'une cellule extra-embryonnaire ayant une ploïdie supérieure à 2 pour la préparation d'un médicament pour une utilisation dans un procédé de diminution du taux d'avortement spontané d'un embryon reconstruit par un mammifère receveur non humain, le procédé comprenant:
la combinaison d'au moins une cellule extra-embryonnaire avec un embryon reconstruit à partir de noyaux non humains et de cellules receveuses non humaines;
le transfert de l'embryon à un mammifère receveur; et
le fait de permettre à l'embryon de se développer en un mammifère post-natal, et à la cellule extra-embryonnaire de se développer en tissu non embryonnaire, diminuant de cette manière le taux d'avortement spontané de l'embryon reconstruit par un animal receveur, à condition que la cellule extra-embryonnaire ne soit pas issue d'un embryon humain.

7. Utilisation selon la revendication 6, dans laquelle la cellule extra-embryonnaire est une cellule tétraploïde.

8. Utilisation selon la revendication 6, dans laquelle la cellule extra-embryonnaire est issue d'un embryon non humain dont les cellules sont fusionnées pour augmenter la ploïdie.

9. Utilisation selon la revendication 6, dans laquelle l'embryon reconstruit est un embryon reconstruit modifié génétiquement.

10. Utilisation selon la revendication 6, dans laquelle l'embryon reconstruit est transféré environ au stade bicellulaire jusqu'au stade de blastocyste.

11. Utilisation d'une cellule extra-embryonnaire ayant une ploïdie supérieure à 2 pour la préparation d'un médicament pour une utilisation dans un procédé de diminution du nombre de défauts chez un mammifère non humain cloné dérivé à partir d'un embryon reconstruit le procédé comprenant:
la combinaison d'au moins une cellule extra-embryonnaire avec un embryon reconstruit à partir de noyaux non humains et de cellules receveuses non humaines;
le transfert de l'embryon à un mammifère receveur non humain; et
le fait de permettre à l'embryon de se développer en un mammifère post-natal, et à la cellule extra-embryonnaire de se développer en un tissu non embryonnaire, diminuant de cette manière le nombre de défauts dans un mammifère non humain cloné issu d'un embryon reconstruit, à condition que la cellule extra-embryonnaire ne soit pas issue d'un embryon humain.

12. Utilisation selon la revendication 11, dans laquelle la cellule extra-embryonnaire est une cellule tétraploïde.

13. Utilisation selon la revendication 11, dans laquelle la cellule extra-embryonnaire est issue d'un embryon non humain dont les cellules sont fusionnées pour augmenter la ploïdie.

14. Utilisation selon la revendication 11, dans laquelle l'embryon reconstruit est un embryon reconstruit modifié génétiquement.

15. Utilisation selon la revendication 11, dans laquelle l'embryon reconstruit est transféré environ au stade bicellulaire jusqu'au stade de blastocyste.

16. Procédé de fabrication d'un mammifère non humain cloné, le procédé comprenant:
la formation d'un embryon reconstruit à partir de noyaux non humains et de cellules receveuses non humaines, étant associé avec au moins une cellule extra-embryonnaire ayant une ploïdie supérieure à 2;
le transfert de l'embryon à un mammifère receveur non humain; et
le fait de permettre à l'embryon de se développer en un mammifère post-natal, et à la cellule extra-embryonnaire de se développer en tissu non embryonnaire, obtenant de cette manière un mammifère non humain cloné, à condition que la cellule extra-embryonnaire ne soit pas issue d'un embryon humain.

17. Procédé de fabrication d'un mammifère non humain transgénique, le procédé comprenant:
l'introduction d'un génome modifié génétiquement d'une cellule donneur mammifère non humaine dans un ovocyte non humain pour former un embryon reconstruit étant associé avec au moins une cellule extra-embryonnaire ayant une ploïdie supérieur à 2; le transfert de l'embryon à un mammifère receveur non humain; et le fait de permettre à l'embryon de se développer en un mammifère post-natal, et à la cellule extra-embryonnaire de se développer en tissu non embryonnaire, fournissant de cette manière un mammifère non humain transgénique, à condition que la cellule extra-embryonnaire ne soit pas issue d'un embryon humain.
